# EUROPEAN PATENT SPECIFICATION

(11) **EP 0 861 669 B1**
(45) Date of publication and mention of the grant of the patent: **29.10.2003**
(21) Application number: 97830593.6
(22) Date of filing: 12.11.1997
(51) Int. Cl.: A61M 1/00, A61B 5/15

(54) **Disposable pediatric set**
Pädiatrieset zum einmaligen Gebrauch
Dispositif pédiatrique à usage unique

(30) Priority: 21.02.1997 IT PA970006
(43) Date of publication of application: 02.09.1998
(73) Proprietor: Target S.r.l., 90017 S. Flavia, (PA) (IT)
(72) Inventor: Bartolone Petra, 900017 S. Flavia (PA) (IT)

(56) References cited:
- WO-A-96/17550
- US-A- 5 000 192
- US-A- 5 059 168
- US-A- 5 575 795

## Description

The invention relates to a disposable pediatric set.

US-A-5 000 192 discloses a prenatal specimen collector in combination with a syringe, wherein the collected blood is filtered upon ejection from the syringe.

The invention provides a disposable pediatric set as defined in the appended claim.

The disposable pediatric set allows to draw from an adult size blood unit a pediatric quantity without effecting the remaining blood.

It permits to supply a pediatric size blood unit, leukocyte depleted, with a filter on line within 24 hours from the blood unit drawing.

The procedure of leukocyte depletion within 24 hours after the blood permits to filter the leukocytes before the hemolysis, avoiding release of viral DNA fragments after having carried out, during 24 hours, the natural bactericide function.

The set allows to reduce the transfusion reaction.

The disposable pediatric set permit repeated use of a given blood unit for the same patient. It is possible with the same cost of one blood bag, to effect two adult size blood unit and one pediatric.

The sterile connection can also be utilized when linking set to blood bag.

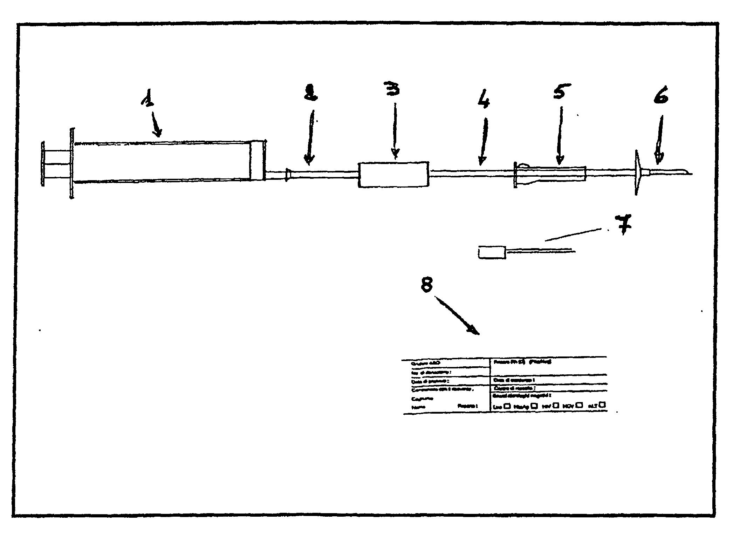

## Claims

1. "A disposable pediatric set for blood drawing from a blood bag or an umbilical cord and preserving a small quantity of filtered blood, the set consisting of a syringe (1), a filter (3), a first (2) and a second (4) tube and a blood drawing needle (6), wherein the syringe (1) has a male Luer lock directly connected by the first tube to an outlet of the filter (3) and an inlet of the filter (3) directly connected by the second tube to the blood drawing needle (6)"

## Patentansprüche

1. Ein einwegpädiatrisches set um Blut aus einer Bluttüte oder Nabelschnur zu entnehmen und dabei eine kleine einer kleinen gefilterte Blutmenge Aufzubewahren. , das Set besteht aus einer Spritze (1), einem Filter (3), einem ersten (2) und zweiten (4) schlauch und einer Nadel für Blutentnahme (6), Eine Spritze (1) hat einen luerlock zapfen von dem ersten schlauch zum filteraus-gang engverbunden (3) und noch einen Filtereingang von dem zweiten schlauch zur Nadel für Blutentnahme (6) engverbunden.

## Revendications

1. Un set pédiatrique mono-utilisation par prélever le sang d'une sac de sang ou du cordon ombilical e garder une petite quantité de sang filtré, la set est composé d'une seringue (1), d'un filtre (3), un premier (2) et une deuxième (4) le tube et une aiguille pour prélever le sang (6), la seringue (1) a un luerlock mâle connecté directement du premier tube à une sortie du filtre (3) et à une entré du filtre (3) directement connecté par le deuxième tube de l'aiguille afin de prélever de sang (6)
